# EUROPEAN PATENT APPLICATION

(11) **EP 2 735 564 A1**
(43) Date of publication of application: **28.05.2014**
(21) Application number: 13194400.1
(22) Date of filing: 26.11.2013
(51) Int. Cl.: C07D 251/54, A61K 8/40, A61Q 17/04

(54) **Stable amorphous solid form of a triazine derivative and the corresponding manufacturing process**

(30) Priority: 27.11.2012 IT MI20122009
(71) Applicant: 3V SIGMA S.p.A, 20121 Milano (IT)
(72) Inventor: Berte', Ferruccio, 24100 BERGAMO (IT); Maestri, Francesco, 24100 BERGAMO (IT); Bemporad, Luca, 24100 BERGAMO (IT); Fabbi, Massimo, 24100 BERGAMO (IT)
(74) Representative: Minoja, Fabrizio

(57) **Abstract**

Disclosed is an amorphous solid form of the compound of formula (I), characterised by an exothermic transition at temperatures lower than 125°C, determined by differential scanning calorimetry (DSC).

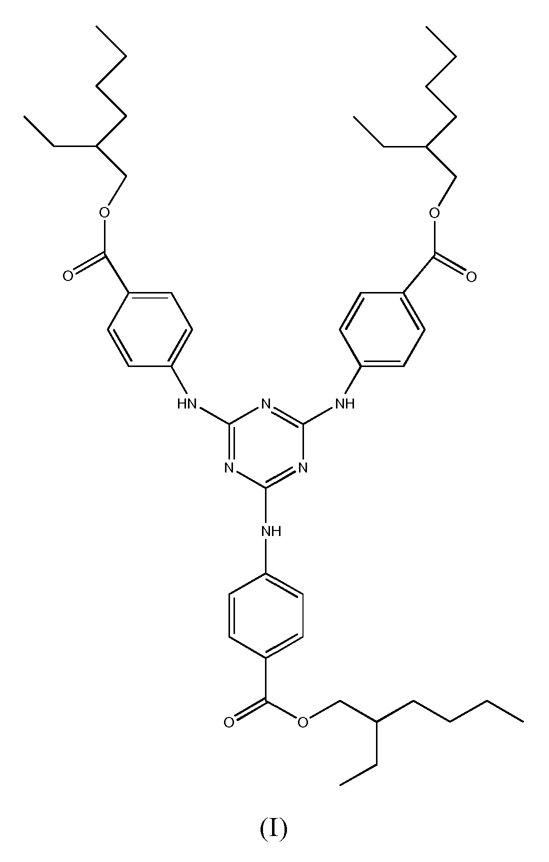

## Description

The present invention relates to the stable amorphous solid form of particular s-triazine derivatives, its manufacturing process and its use as a sunscreen or light stabilizer.

### Prior art

Ultraviolet solar radiation has a damaging effect on the skin tissue, and causes the degradation of polymers. By using particular compounds, called sunscreens, which absorb the UV part of solar radiation, harmful effects and aging of the skin and polymer materials can be prevented, or at least slowed.

Numerous substances have been studied and tested as protective agents, and a great deal of patent literature now exists relating to compounds belonging to various chemical classes that absorb in the ultraviolet region, particularly radiation between 290 and 320 nm, called UV-B, which is very harmful.

Relatively few of these compounds have proved suitable for practical application. They include p-methoxycinnamic acid and p-dimethylaminobenzoic acid esters, benzotriazoles and hydroxybenzophenones.

A drawback shared by all these compounds is their low ability to absorb radiation between 290 and 320 nm, which means that relatively large quantities are required to obtain the optimum photoprotective effect.

An excellent UV-B absorber should have the following characteristics:
1) High specific extinction at 290-320 nm allowing the use of low doses, resulting in cost savings and minimal toxicological risk
2) Light stability
3) Heat stability
4) Oxidation stability
5) Stability to different pHs
6) Good solubility in the basic substances commonly used for dermatological formulations
7) Negligible toxicity
8) Colour and odour compatibility with the intended applications
9) High molecular weight, which reduces the probability of absorption by the skin and increases toxicological safety
10) Compatibility with the different substances generally used in dermatological formulations.

US 4617390 and US 4724137 disclose s-triazine derivatives obtained by reacting trichlorotriazine with p-amino-benzoic acid esters, which absorb intensely in the UV-B zone. Unfortunately, the solubility of these compounds in the solvents generally used to formulate sun creams is very low, which makes their practical use problematic and very difficult, especially when the percentage of photoprotector in the composition must be increased to prepare formulations with a high sun protection factor.

One of these compounds in particular, namely 2,4,6-trianilino-p-(carbo-1'-ethylhexyl-1'-oxy)-1,3,5-triazine, also known as octyl triazone, is widely used and has obtained authorisations on the market as a photoprotector of formulations and the skin in the field of cosmetic sunscreens. This compound is identified by the CAS number 88122-99-0, has the INCI name ethylhexyl triazone, and is known on the market under various names, including Uvinul T 150 (Basf) and Uvasorb ET (3V Sigma Spa).

The structural formula of said compound is:

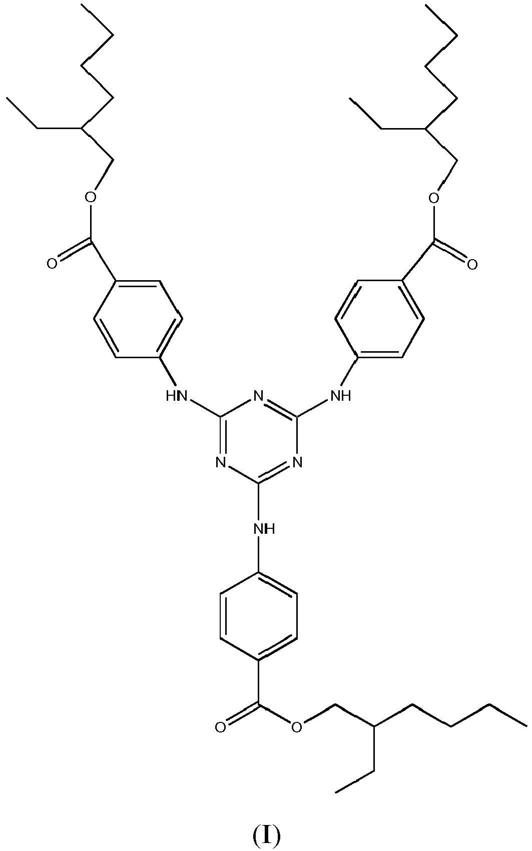

Over the years, some attempts have been made to improve the solubility of said compound in the solvents and oils generally used in cosmetic formulations.

US4656272 discloses the preparation of said compound in the presence of esters of branched alkanoic acids, and its isolation in mixtures with them.

US6531117 discloses stable aqueous dispersions of various sunscreens including stable aqueous colloidal dispersions of said compound in its amorphous or partly amorphous form, divided at microscopic level and obtained by particular precipitation techniques from solvents in the presence of protective colloids.

US7074922 discloses the preparation of a tautomeric form of said compound by a particular process of precipitation of its tautomeric forms from particular mixtures of solvents.

The stable vitreous amorphous form of said pure compound and its tautomeric forms is unknown.

### Description of the invention

We have surprisingly found that the compound of formula (I) can easily be obtained, still pure, in its amorphous solid or non-crystalline form by rapid cooling of its molten mass or rapid elimination of the solvent from a solution thereof at temperatures lower than its melting point.

The amorphous solid form of compound (I) is stable at normal storage temperatures for long periods, and can easily be ground to a powder that is not caked, and is easy to use.

The amorphous form of compound (I) in the form of powder, granules or flakes is much more rapidly dissolvable in common solvents and cosmetic oils than the corresponding crystalline form, which is already known and identifiable by the existence of a precise melting point, generally between 126 and 132°C.

We have also found that in many cases, unlike the known crystalline form, the amorphous solid form is soluble in reasonable times in cosmetic oils at much lower temperatures, and even at room temperature.

The amorphous solid form of compound (I) is easily identifiable by thermal analysis techniques such as differential scanning calorimetry (known as DSC).

With this technique, it can be seen that the amorphous solid form presents an exothermic transition at temperatures of between 50 and 110, typically from 50 to 90°C, i.e. much lower than the endothermal transition temperatures of the melting point of the corresponding crystalline form. The values of the two transition areas are comparable, demonstrating that the substance obtained is almost totally amorphous.

Compound (I) in its known crystalline form does not present any exothermic transition at low temperature, only those relating to melting close to or at the melting point.

The first subject of the invention is therefore the amorphous solid form of the compound of formula I, characterised by exothermic transition at temperatures below 125°C, and generally between 50 and 110°C, determined by differential scanning calorimetry (DSC). The invention presents as a solid with a vitreous appearance.

The amorphous solid form can coexist with the known crystalline form.

However, the amorphous solid form according to the present invention is preferably present in a proportion exceeding 10% by weight, more preferably exceeding 40%, and even more preferably exceeding 80% by weight.

The amorphous solid form of compound (I) can also be characterised by other analysis techniques, such as X-ray diffractometry and density measurements.

The amorphous solid form of compound (I) is obtained easily and efficiently by rapidly cooling, or supercooling, in under a few seconds or minutes, the compound melted at temperatures exceeding 126-132°C, to temperatures below 60°C, preferably below 40°C, and even more preferably below 30°C.

It is preferable to generate the amorphous form in times of less than two hours, preferably less than 20 minutes, more preferably less than 5 minutes, and even more preferably less than 2 seconds.

This process can be performed, for example, by pouring the molten compound onto the cold surface of a suitably cooled flaking machine or into a cold, inert non-solvent such as water, methanol, ethanol or n-heptane.

Slower cooling which lasts several hours can achieve the same purpose, but is usually uneconomical.

The amorphous solid form of compound (I) can also be obtained from a solution thereof in solvent by rapidly removing the solvent, in particular at temperatures below melting point, for example with the aid of a vacuum or flow of air or inert gas such as nitrogen.

In many cases it can be useful to employ rapid cooling techniques such as spray-cooling, or rapid liquid removal techniques such as spray-drying or fluid-bed drying. In these cases the solidification times of the product can be as short as fractions of a second.

Alternatively, a solution of compound (I) is poured into a non-solvent system so that the substance precipitates and can be recovered at temperatures below melting point by known methods such as filtration and drying.

The solvents that can be used include alcohols such as for example ethanol, propanol, isopropanol, butanol, isobutanol, hexanol, 2-ethylhexanol, octanol and dodecanol; glycols such as propylene glycol; ketones such as acetone, methyl ethyl ketone and methyl isobutyl ketone; esters such as ethyl acetate and butyl acetate; aliphatic and aromatic hydrocarbons such as petrols, mineral spirits, benzene, toluene and xylene.

The non-solvents that can be used include for example water, methanol, heptane and decane.

Compound (I) can be synthesized by the techniques already known and described in US 4617390 and US 4724137 cited above by reacting cyanuryl chloride with 2-ethylhexyl p-aminobenzoate in a solvent as hydrocarbon units or xylene. The crystalline form of compound (I) is obtained by recrystallisation.

The amorphous solid form according to the present invention can also be produced in these cases from the solution of compound (I) obtained at the end of the reaction without performing the subsequent recrystallisation stages, but first eliminating the solvent and rapidly cooling the molten substance obtained or rapidly eliminating the solvent in short times at temperatures below melting point.

The amorphous solid form can easily be produced by melting the crystalline form at temperatures higher than its melting point (126-132°C), followed by rapid cooling.

The amorphous solid form can also be obtained by dissolving the crystalline form in a solvent thereof and rapidly eliminating the solvent at temperatures below melting point.

The amorphous solid form of compound (I) according to the invention can be advantageously used in the form of powder, granules or flakes in plastics, coatings, cosmetic formulations, and in particular in sunscreens.

Many cosmetic formulations consist of emulsions containing an oil phase. In particular, cosmetic formulations containing sunscreens such as the compound of formula (I) are generally of this type. The method most commonly used for their manufacture requires the filter to be dissolved first in the oil phase, followed by production of the emulsion. The dissolution rate of the sunscreen and the temperature at which said dissolution takes place in reasonable times are therefore important economic factors.

The amorphous solid form according to the invention can be dissolved easily in many emollients and polar oils typically used in the cosmetic field, especially in sunscreen formulations. Examples of said oils are (INCI Names): Hexyl Laurate, C12-13 Alkyl Lactate, PPG-3 Myristyl Ether, Propylene Glycol Monoisostearate, Di-C12-13 Alkyl Malate, C 12-13 Alkyl Octanoate, Cocoglycerides, Tridecyl Salicylate, Di-C12-13 Alkyl Tartrate, PEG-7 Hydrogenated Castor Oil, Dioctyl Adipate, Octyldodecanol, PPG-2 Myristyl Ether Propionate, Propylene Glycol Dicaprylate/ Dicaprate, Isopropyl PPG-2 Isodeceth-7-Carboxylate, PEG-7 Glyceryl Cocoate, Diisopropyl Adipate, Cetearyl Isononanoate, Coco Caprylate/Caprate, Dicaprylyl Maleate, Diethylhexyl Malate, Ethylhexyl Cocoate, Ethylhexyl Ethylhexanoate, Ethylhexyl Isostearate, Ethylhexyl Methoxycinnamate, Ethylhexyl Palmitate, Ethylhexyl Salicylate, C12-C15 Alkyl Benzoate, Caprylic/Capric Triglyceride, Isopropyl Myristate, Isopropyl Palmitate, Isopropyl Stearate, Ethylhexyl Stearate, Ethylhexyl Benzoate and Propylene Glycol Dicaprylate/Dicaprate.

The amorphous solid form according to the invention can also be dissolved in all its typical solvents, such as for example alcohols including ethanol, propanol, isopropanol, butanol and 2-ethylhexanol, glycols such as propylene glycol, esters such as ethyl acetate and butyl acetate, ethers such as tetrahydrofuran, ketones such as acetone, methyl ethyl ketone and methyl isobutyl ketone, and aliphatic and aromatic hydrocarbons such as toluene and xylenes.

The amorphous solid form according to the present invention can also be dissolved in other UVA and UVB sunscreens in the liquid state.

Within the narrower solubility ranges applicable in various cases, the amorphous solid form according to the present invention can also be used in less polar oils, such as liquid paraffin and dicaprylyl ether.

In particular, the amorphous solid form according to the invention can be advantageously introduced into formulas for cosmetics, either as the only sunscreen or in combination with other known sunscreens, instead of forms obtained by known processes.

These formulations constitute a second subject of the invention. Said formulations will preferably contain one or more conventional UVA and UVB sunscreens such as those listed in Annex VII to the European Cosmetics Directive (76/768/EEC). Even more preferably, the formulations may contain, in addition to the amorphous solid form according to the invention, one or more sunscreens selected from 2-ethylhexyl p-methoxycinnamate, 2-hydroxy-4-methoxybenzophenone, 2-hydroxy-4-methoxybenzophenone-5-sulphonic acid, 3-(4'-methylbenzylidene)-d,l-camphor, diethylhexyl butamido triazone, 4-(tert-butyl)-4'-methoxy-dibenzoylmethane, 2-cyano-3,3-diphenylacrylic acid 2-ethylhexyl ester, bis-ethylhexyloxyphenol-methoxyphenyl-triazine, methylene-bis-benzotriazolyl-tetramethylbutylphenol, benzoic acid 2-(4-diethylamino-2-hydroxybenzoyl)-hexyl ester, titanium dioxide and zinc oxide.

Thermal analysis of the samples (DSC) were performed using a Perkin-Elmer differential scanning calorimeter, model DSC-7. Samples were closed in 30 microliter aluminium pans to perform the analysis.

Thermal analysis were done using the following thermal profile: from 45°C to 115°C at 10°C/min. heating rate, then 1 minute isothermal step at 115°C, from 115° to 135°C at 1°C/min. heating rate.

The examples below illustrate the invention in greater detail.

### Example 1

100 g of Uvasorb ET (3V Sigma Spa) in the form of a white crystalline powder (Sample 1C) with a melting point of 128°C, corresponding to the compound of formula (I), was melted at 150°C to obtain a slightly viscous liquid, and poured rapidly onto a flat PTFE tray at 25°C. Within 5 minutes a fragile vitreous mass formed, which was ground and sieved below 200 microns, to obtain a white powder (Sample 1A).

Samples 1C and 1A were characterised by the differential scanning calorimetry (DSC) technique. Sample 1C did not present any thermal transition until the expected known endothermic melting peak at 128°C. The amorphous solid sample 1A according to the present invention presented a first exothermic transition (corresponding to a formation of crystalline form) at temperatures of between 50 and 110°C, and subsequently at 128°C, the endothermic transition corresponding to melting of the crystalline form that formed at lower temperatures. The areas of the two curves, corresponding to the energies involved in the transitions, were comparable.

Sample 1A was stored for 12 months at room temperature and subjected to a second DSC measurement. Its appearance was still that of a white powder. The DSC curve of this sample, aged for 12 months, still presented the exothermic peak between 50 and 110°C, and was identical to the curve obtained a year earlier.

Sample 1A is therefore an amorphous, solid, stable form of the compound of formula (I).

### Example 2

Example 1 was repeated using 100 g of Uvinul T 150 (Basf) in the form of a white crystalline powder (Sample 2C) to obtain the corresponding amorphous solid form (Sample 2A).

Once again, the DSC technique demonstrated that sample 2A was a stable amorphous form of the compound of formula (I).

### Example 3

Example 1 according to the present invention was repeated using 100 g of compound (I) with melting point 128°C obtained in accordance with the experimental conditions described in example 1 of US 4617390. The compound was melted at 140°C followed by rapid cooling on a cold surface to 25°C to obtain the amorphous solid form, which was ground and sieved below 200 microns (Sample 3A).

Once again, the DSC technique demonstrated that sample 3A was a stable amorphous form of the compound of formula (I).

### Example 4

10 g of Uvasorb ET was dissolved in 10 ml of acetone and dried under vacuum at a maximum of 30°C for 20 minutes. The vitreous mass obtained was ground and sieved below 200 microns to obtain sample 4A in amorphous solid form.

### Application example 5

### Description of dissolution rate test.

4.0 g of powder was dispersed under stirring in 96.0 g of caprylic/capric triglyceride solvent at 25°C.

The total dissolution time was measured, with visual inspection, noting the time of complete disappearance of any heterogeneity in the form of particles or opalescence in the solution.

### Results.

| | |
|---|---|
| Sample 1A dissolution time | 15 minutes |
| Sample 2A | 15 minutes |
| Sample 3A | 15 minutes |
| Sample 1C | over 90 minutes |
| Sample 2C | over 90 minutes |

### Application example 6

### Description of dissolution rate test.

4.0 g of powder was dispersed under stirring in 96.0 g of ethylhexyl stearate solvent at 25°C.

The total dissolution time was measured, with visual inspection, noting the time of complete disappearance of any heterogeneity in the form of particles or opalescence in the solution.

### Results.

| | |
|---|---|
| Sample 1A | dissolution time 40 minutes |
| Sample 1C | over 420 minutes (opaque white dispersion) |

### Application example 7

### Description of dissolution rate test.

4.0 g of powder was dispersed under stirring in 96.0 g of octyl dodecanol solvent at 25°C.

The total dissolution time was measured, with visual inspection, noting the time of complete disappearance of any heterogeneity in the form of particles or opalescence in the solution.

### Results.

| | |
|---|---|
| Sample 1A | dissolution time 90 minutes |
| Sample 1C | over 420 minutes (insoluble - opaque white dispersion) |

### Application example 8

Amorphous solid sample 1A according to the present invention was used to prepare two cosmetic formulas of oil-in-water emulsion (cosmetic formula 1 and cosmetic formula 2). The sun protection factor was measured *in vitro* (SPF *in vitro*) on said two formulas with a Labsphere UV-2000S instrument, in the UV-visible zone from 290 to 400 nm. For the experimental measurement of the SPF the cosmetic formula was applied to Transpore tape (3M Inc.) at the concentration of 2.0 mg/cm². 3 tapes were prepared for each formula, 12 readings being taken per tape, and readings with covariance >10% above the average were rejected.

The following mean values were obtained:

| | |
|---|---|
| Cosmetic formula 1 | SPF *in vitro* = 4.9 |
| Cosmetic formula 2 | SPF *in vitro* = 14.2 |

### Cosmetic formula 1

Preparation: phase I and phase II are heated separately, under stirring, at 70-75°C, until the ingredients are completely solubilised. Phase II is added to phase I, maintaining the same temperature and emulsifying with a Silverson homogeniser at 3000 rpm. After the addition of phase III, the preparation is cooled to 40°C. After the addition of phase IV, the product obtained is discharged.

| **Phase** | **Ingredient** | **INCI name** | **% (w/w)** |
|---|---|---|---|
| **I** | Cremophor GS32 | Polyglyceryl-3 Distearate | 3 |
| | Crodet S40 | PEG-40 Stearate | 0.3 |
| | Lanette O | Cetearyl Alcohol | 2 |
| | Cutina GMS | Glyceryl Stearate | 1 |
| | Cetiol OE | Dicaprylyl Ether | 7.5 |
| | Ceraphyl 230 | Diisopropyl Adipate | 10.5 |
| | Sample 1A | Ethylhexyl Triazone | 3 |
| **II** | Demineralised water | Water | up to 100 |
| | EDTA | EDTA | 0.1 |
| | Synthalen K | Carbomer | 0.15 |
| | Propylene glycol | Propylene Glycol | 3 |
| **III** | Triethanolamine | TEA | 0.2 |
| **IV** | Microcare PM5 | Methyl paraben, Ethyl paraben, Propyl paraben, Butyl paraben, Isobutyl paraben, 2-phenoxyethanol | 0.5 |

### Cosmetic formula 2

Preparation: phase I and phase II are heated separately, under stirring, at 70-75°C, until the ingredients are completely solubilized. Phase II is added to phase I, maintaining the same temperature and emulsifying with a Silverson homogeniser at 3000 rpm. After the addition of phase III, the preparation is cooled to 40°C. After the addition of phase IV, the product obtained is discharged.

| **Phase** | **Ingredient** | **INCI name** | **% (w/w)** |
|---|---|---|---|
| **I** | Simusol 165 | Glyceryl Stearate (and) PEG-100 Stearate | 2 |
| | Lanette O | Cetearyl Alcohol | 0.5 |
| | Cetiol AB | C12-15 Alkyl Benzoate | 18 |
| | Sample 1A | Ethylhexyl Triazone | 2 |
| | Eusolex 9020 | Butyl Methoxydibenzoylmethane | 5 |
| | Eusolex OCR | Octocrylene | 5 |
| | Ueusolex 2292 | Ethylhexyl Methoxycinnamate | 0.1 |
| **II** | Demineralised water | Water | up to 100 |
| | Glycerin | Glycerin | 3 |
| | Synthalen K | Carbomer | 0.15 |
| **III** | Triethanolamine | TEA | 0.2 |
| **IV** | Microcare PM5 | Methyl paraben, Ethyl paraben, Propyl paraben, Butyl paraben, Isobutyl paraben, 2-phenoxyethanol | 0.5 |

## Claims

1. Amorphous solid form of the compound of formula (I) **characterised by** an exothermic transition at temperatures ranging from 50 to 110°C as determined by differential scanning calorimetry (DSC).

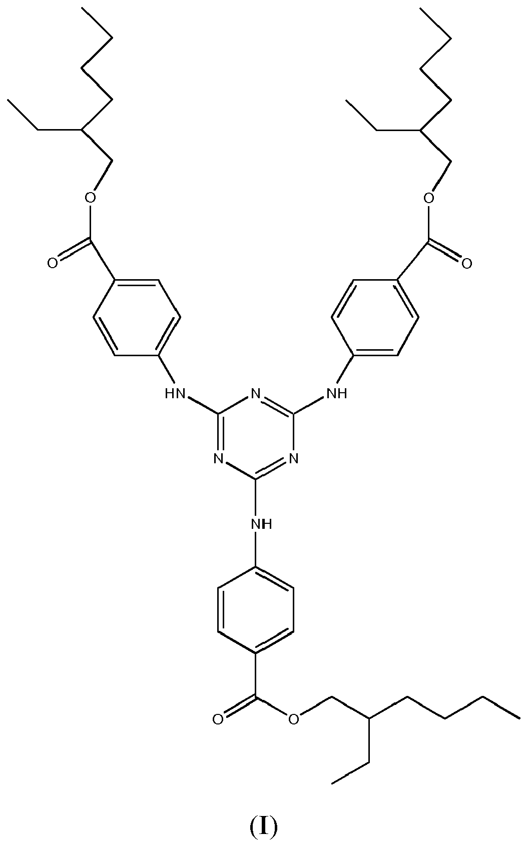

2. Amorphous solid form of the compound of formula (I) according to claim 1 **characterised by** an exothermic transition at temperatures ranging from 50 to 90°C

3. Solid form according to claim 1 or 2 wherein the amorphous part is present in an amount higher than 10% by weight, preferably higher than 40% by weight and preferably higher than 80% by weight.

4. Process for the production of the amorphous solid form of claims 1-3 by rapid cooling of the molten compound (I) on a cold surface or in a cold fluid.

5. Process according to claim 4 wherein the fluid is selected from nitrogen, air or a non-solvent selected from water, alcohol and heptane.

6. Process for the production of the amorphous solid form of claims 1-3 by rapid elimination of the solvent from a solution of compound (I) at temperatures lower than the melting point.

7. Process according to claim 6 wherein the solvent is an alcohol, a ketone, an ester or a hydrocarbon.

8. Process according to claim 7 wherein the solvent is an aliphatic C4-C12 alcohol.

9. Process according to claim 8 wherein the solvent is an octanol, in particular 2-ethylhexanol.

10. Use of the amorphous solid form of claims 1-3 as powder, granules or flakes in plastics, coatings or cosmetic formulations, and in particular in sunscreens.

11. Use of the amorphous solid form according to claim 10 as powder, granules or flakes in combination with additional known UVA and UVB sunscreens.

12. Use according to claim 11 wherein the UVA and UVB sunscreens are selected from 2-ethylhexyl p-methoxycinnamate, 2-hydroxy-4-methoxybenzophenone, 2-hydroxy-4-methoxybenzophenone-5-sulphonic acid, 3-(4'-methylbenzylidene)-d,l-camphor, diethylhexyl butamido triazone, 4-(tert-butyl)-4'-methoxy-dibenzoylmethane, 2-cyano-3,3-diphenylacrylic acid 2-ethylhexyl ester, bis-ethylhexyloxyphenol-methoxyphenyl-triazine, methylene-bis-benzotriazolyl-tetramethylbutylphenol, benzoic acid 2-(4-diethylamino-2-hydroxybenzoyl)-hexyl ester, titanium dioxide and zinc oxide.

13. Cosmetic formulations comprising the amorphous solid form of claims 1-3, optionally in admixture with additional UVA or UVB filters.

14. Cosmetic formulations according to claim 13 wherein the additional UVA or UVB filters are selected from 2-ethylhexyl p-methoxycinnamate, 2-hydroxy-4-methoxybenzophenone, 2-hydroxy-4-methoxybenzophenone-5-sulphonic acid, 3-(4'-methylbenzylidene)-d,l-camphor, diethylhexyl butamido triazone, 4-(tert-butyl)-4'-methoxy-dibenzoylmethane, 2-cyano-3,3-diphenyl-acrylic acid 2-ethylhexyl ester, bis-ethylhexyloxyphenol-methoxyphenyl-triazine, methylene-bis-benzotriazolyl-tetramethylbutylphenol, benzoic acid 2-(4-diethylamino-2-hydroxybenzoyl)-hexyl ester, titanium dioxide and zinc oxide.

15. Method for the preparation of cosmetic formulations which comprises solubilisation of the amorphous form of claims 1-3 in a cosmetic oil.
